Europäisches Patentamt

(19) European Patent Office

Office européen des brevets

(11) **EP 0 600 915 B1**

(12) **FASCICULE DE BREVET EUROPEEN**

(45) Date de publication et mention
de la délivrance du brevet:
**12.11.1997 Bulletin 1997/46**

(21) Numéro de dépôt: **92915740.2**

(22) Date de dépôt: **03.07.1992**

(51) Int. Cl.$^6$: **A61K 35/78**

(86) Numéro de dépôt international:
**PCT/FR92/00629**

(87) Numéro de publication internationale:
**WO 93/00916 (21.01.1993 Gazette 1993/03)**

(54) **COMPOSITION A BASE DE BUIS POUR LE TRAITEMENT DE L'INFECTION PAR LE VIH (VIRUS DE L'IMMUNODEFICIENCE HUMAINE)**

BUCHSBAUM ZUBEREITUNG FÜR DIE BEHANDLUNG VON HIV INFEKTION

BOX-BASED COMPOSITION FOR TREATING HIV (HUMAN IMMUNODEFICIENCY VIRUS) INFECTION

(84) Etats contractants désignés:
**AT BE CH DE DK ES FR GB GR IT LI LU MC NL SE**

(30) Priorité: **10.07.1991 FR 9108790**

(43) Date de publication de la demande:
**15.06.1994 Bulletin 1994/24**

(73) Titulaires:
• **UNIVERSITE DE NICE-SOPHIA ANTIPOLIS**
**F-06100 Nice (FR)**
• **DURANT, Jacques**
**F-06000 Nice (FR)**
• **DELLAMONICA, Pierre**
**F-06000 Nice (FR)**
• **REBOUILLAT, Alain**
**06100 Nice (FR)**

(72) Inventeurs:
• **DURANT, Jacques**
**F-06000 Nice (FR)**
• **DELLAMONICA, Pierre**
**F-06000 Nice (FR)**
• **REBOUILLAT, Alain**
**69, avenue Durandy**
**F-06100 Nice (FR)**

(74) Mandataire:
**Warcoin, Jacques et al**
**Cabinet Régimbeau,**
**26, avenue Kléber**
**75116 Paris (FR)**

(56) Documents cités:
**GB-A- 782 469**

**Description**

L'invention a pour objet une composition à base de buis pour le traitement du syndrome d'immuno-dépression acquis (S.I.D.A) ainsi que les patients séropositifs pour ce rétrovirus ainsi que toutes les pathologies dans lesquelles le TNF (Facteur de nécrose des tumeurs ou Tumour Necrosis Factor) peut être impliqué directement et indirectement.

L'état de la technique peut être défini par les activités pharmacodynamiques connues à ce jour du buis : Buxus sempervirens L : BUXACEES et par les nombreux brevets décrivant l'utilisation d'extrait comportant desalcaloïdes et en particulier de 17-cetostéroïdes ou non pour fabriquer un médicament pour le traitement et la prévention des infections rétrovirales.

Depuis l'antiquité, les extraits des parties du buis, surtout des feuilles, de l'écorce et des branches présentent certaines propriétés pharmacodynamiques.

Les feuilles étaient utilisées comme laxatifs, l'écorce comme agent sudorifique et fébrifuge, les graines comme astringent et tonique.

Le brevet FR-A-2.425.241 décrit un produit capillaire pour arrêter la chute des cheveux.

Le brevet FR-N° 3.114 M décrit un médicament à base d'extrait ou de corps actifs extraits du buis qui renforce la puissance du muscle cardiaque et ayant une action stimulante sur le coeur.

- FR-A-2.615.394 : ce brevet décrit l'utilisation de certains 17 cétostéroïdes, éventuellement avec un immunomodulateur et/ou un agent antiviral, pour fabriquer un médicament pour le traitement et la prévention des infections rétrovirales.
- EP 0394.112 : ce brevet décrit l'utilisation de dipyrido (4,3-b) (3,4-f) indoles pour la préparation de médicaments utiles pour le traitement du SIDA.
- EP 0419.861 A2 : ce brevet décrit le dibenzo b,f 1,4 oxazépine (et thiazépine)-11 (10H)-ones et -thiones et leur utilisation pour la prévention ou le traitement du SIDA.
- EP 0417.534 A1 : ce brevet décrit le pyrido 2,3-b 1,4 benzoxazépine- (et -thiazépine)-6(5H)-ones et -thiones et leur utilisation pour la prévention ou le traitement du SIDA.
- GB-A-782.469 : ce brevet décrit des compositions à base de Buxus Sempervirens aptes à combattre la tuberculose ainsi que les procédés pour les obtenir.

L'invention a pour objet l'application d'une plante de la famille des buxacées pour la préparation d'un médicament destiné au traitement préventif et/ou curatif du SIDA ou de syndromes analogues ainsi que le traitement des patients séropositifs pour ce rétrovirus.

L'invention a pour objet une composition pour le traitement de déficit immunitaire en corrigeant les paramètres actuellement utilisés pour définir cette pathologie par le fait qu'elle utilise une plante de la famille des buxacées.

Un autre objet de l'invention est d'associer un principe actif destiné au traitement du SIDA, ou de syndromes analogues, et une composition contenant une plante de la famille des buxacées à l'état pur ou en combinaison avec des excipients inertes et pharmaceutiquement acceptables.

La principale espèce de buxacées est le buis : Buxus sempervirens.

La partie utilisée est essentiellement la feuille de la plante.

La partie utilisée est également la tige (bois), la racine et l'écorce de la racine.

La composition est notamment constituée d'alcaloïdes stéroïdiques spécifiques de la famille des buxacées dérivés du cycloarténol : cyclobuxine D, buxamine notamment.

De nombreux livres et articles déjà parus concernent la constitution du buxus, dont les principales références bibliographiques sont :

- ATTA-UR-RAHMAN, AHMED-D, ASIF-E, AHMAD-S, SENER-B, TURKOZ-S. CHEMICAL CONSTITUENTS OF BUXUS-SEMPERVIRENS J-NAT-PROD (LLOYDIA) 54 (1).1991.79-82
- RAHMAN-A-U, AHMED-D, IQBAL-CHOUDHARY-M, TURKOZ-S, SENER-B. CHEMICAL CONSTITUENTS OF BUXUS-SEMPERVIRENS. PLANTA-MED 54(2).1988.173-174.
- RAHMAN-A, ASIF-E, AHMED-D, SENER-B, TURKOZ-S. HEJ RESEARCH INSTITUTE OF CHEMISTRY, UNIVERSITY OF KARACHI, KARACHI-32 PAK.
  NEW ALKALOIDS FROM BUXUS SEMPERVIRENS
  J-NAT-PROD-LLOYDIA, 1989, VOL/ISS/PG. 52/6 (1319-1322), ISSN:0163-3864

L'invention a également pour objet l'application d'une plante de la famille des buxacées pour la préparation d'un médicament destiné au traitement des affections dans lesquelles intervient le TNF.

La posologie utilisée est d'environ 330 mg toutes les 8 heures sous forme de poudre et administrée en gélules.

Cette poudre est obtenue par passage au mixeur, la taille des particules obtenues est d'environ 50 μm. Le broyage est fait de manière intermittente pour éviter tout échauffement susceptible d'altérer le ou les produits actifs. La poudre est alors mise dans des gélules qui sont stockées au sec dans des sachets de 50 gélules.

La dose toxique inscrite au Codex en 1974 et 1979 serait supérieure à 4 grammes pour la teinture et 10 g en décoction. La posologie choisie est donc en deça des valeurs susceptibles d'être toxique.

En décembre 1990, les inventeurs ont observé qu'une des patientes présentait des caractéristiques immunitaires non compatibles avec son stade dans la classification du CDC.

Cette patiente, en effet, a présenté une pneumocystose pulmonaire en Août 1989 d'évolution favorable sous traitement adapté. Celle-ci a retenu l'attention des inventeurs, car dix huit mois après cette première infection opportuniste, son taux de CD4 était anormalement élevé compte-tenu de l'évolution naturelle connue de cette maladie (la moyenne de survie de cette infection opportuniste étant de quatorze mois lors de sa contamination).

Au décours d'une consultation prévue, il a été réalisé un interrogatoire rétrospectif "policier" qui a permis de mettre en évidence que ladite patiente ingérait régulièrement depuis cette époque, de nombreuses plantes.

Dans un premier temps, il a été établi la chronologie de la prise de ces différentes plantes par rapport à l'évolution des CD4 et il a été constaté, après étude des résultats, une corrélation entre la remontée des CD4 et la prise de buis.

Cette plante répertoriée dans le Codex est connue pour un certain nombre d'effets toxiques à partir d'une certaine dose. Ceci a donc permis de définir de façon empirique une dose dont il est possible d'espérer un effet thérapeutique mais dénuée de tout effet toxique.

Des gélules ont été fabriquées pour continuer de façon rationnelle le traitement.

LES FAITS :

- patiente, de sexe féminin
- sérologie VIH (virus de l'immunodéfcicience humaine) positive découverte en Août 1986
- interruption thérapeutique de grossesse en 1988 et 1989
- toxicomanie intraveineuse à l'héroïne débutée en 1982 et stoppée transitoirement en 1985. Depuis, prise intermittente de drogues.
- date probable de contamination : 1980-1985
- autres antécédents médicaux : syndrome dépressif avec 3 tentatives de suicides en 1982. Hépatite virale B en 1983.
- 8/89 : hospitalisation pour altération de l'état général avec fébricule à 38, toux. Découverte d'une pneumocystose au lavage bronchoalvéolaire traitée par aérosol de lomidine pendant 21 jours. Evolution clinique favorable.
- 8/89 : mise sous traitement par Zidovudine (Rétrovir marque déposée) à la posologie de 300 mg toutes les 8 heures. En raison d'une intolérance digestive, ce traitement a été stoppé volontairement par la patiente un mois plus tard.
- à partir de cette date, la patiente est suivie mensuellement en hôpital de jour. L'évolution de la maladie est sans particularités hormis un syndrome dépressif chronique ; l'état général étant parfaitement conservé. Aucune autre infection opportuniste et secondaire n'a été observée durant cette période.
- l'évolution de ces paramètres immunologiques (CD4 - CD8 - CD3/ mm3 et Antigénémie P24) est résumée dans le tableau suivant :

| MOIS | Lympho | CD4 | CD8 | CD3 | Prise de X |
|---|---|---|---|---|---|
| SUIVI RETROSPECTIF | | | | | |
| Juin 88* | 1044 | 450 | 476 | ? | |
| Mai 89* | 1027 | 256 | 400 | ? | |
| Août 89** | 1089 | ? | ? | ? | 0 |
| Oct 89 | 2046 | 695 | 941 | 1677 | +++ |
| Déc 89 | 1380 | 317 | 759 | 1104 | +++ |
| Janv 90 | 1652 | 648 | 900 | 1566 | 0 |
| Fev 90 | 1450 | 478 | 623 | 1174 | ++ |
| Mars 90 | 1650 | 445 | 808 | 1270 | ++ |
| Mai 90 | 1580 | 354 | 646 | 1047 | + |
| Juin 90 | 1350 | 378 | 540 | 958 | +++ |
| Juillet90 | 1690 | 659 | 709 | 1368 | +++ |
| Sept 90 | 1430 | 214 | 829 | 1086 | + |
| Oct 90 | 1690 | 388 | 912 | 1335 | 0 |
| Dec 90 | 1580 | 489 | 695 | 1200 | + |
| Janv 91 | 1540 | 323 | 908 | 1278 | ++ |
| Fév 91 | 1240 | 322 | 669 | 992 | 0 |
| SUIVI PROSPECTIF | | | | | |
| 6 mars 91 | 1120 | 112 | 594 | 852 | ++++ |
| 18 mars91 | 1370 | 238 | 896 | 1162 | ++++ |
| 3 avr 91 | 1625 | 373 | 1040 | 1088 | ++++ |
| 16 avr 91 | 2510 | 660 | 1175 | 1876 | ++++ |
| 30 avr 91 | 2260 | 689 | 1160 | 1853 | ++++ |

\* Interruption volontaire et thérapeuthique de grossesse
\*\* Pneumocystose pulmonaire prouvée à la fibroscopie avec lavage bronchoalvéolaire

Lors du suivi prospectif, une corrélation entre la quantité de prise de buis ingérée et l'augmentation de CD4 a été constatée par les inventeurs. En effet, celle ci a arrêté spontanément la prise de buis à partir du 15 février 1991 jusqu'au 6 mars 1991. Dès lors une chute importante des CD4 (110/mm3) a été observée.

A partir de cette date, la patiente a repris de manière régulière les gélules et les inventeurs ont observé une remontée quasi-linéaire des lymphocytes CD4.

Au 30 avril 1991, la patiente présentait un taux de CD4 dans les limites inférieures de la normale. Par ailleurs, l'état général de la patiente s'est amélioré avec une reprise pondérale de 4 kilos en l'espace de deux mois.

Durant cette période, aucune autre thérapeutique particulière n'était prescrite. Le suivi prospectif des paramètres immunitaires est représenté sur les schémas suivants :

Les graphiques ci-joints sont donnés à titre d'exemples indicatifs et non limitatifs. Ils représentent un mode de réalisation préféré selon l'invention. Ils permettront de comprendre aisément l'invention.

La figure 1 est une vue schématique mettant en évidence l'évolution des CD4.

La figure 2 est une vue schématique mettant en évidence l'évolution des CD3.

La figure 3 est une vue schématique mettant en évidence l'évolution des CD8.

La figure 4 est une vue schématique mettant en évidence l'évolution des lymphocytes.

MISE EN EVIDENCE DE L'ACTIVITE ANTIVIRALE
(extraction hydroalcoolique Ethanol 80 % Eau 20 %)

L'activité anti-HIV a été déterminée :

1°) par la mesure de l'inhibition de l'effet cytopathogène (CPE) et de la production de la transcriptase inverse (RT) sur des cellules lymphoblastoïdes CEM-clone 13 infectées par VIH.

Mesure de l'inhibition de l'effet cytopathogène (CPE) :

Le produit mis en solution dans la N-méthyl pyrrolidone, est étudié à différentes concentrations (0,015 à 50 $\mu$g/ml). Avant l'infection, les cellules sont incubées avec ou sans produit pendant 1 heure à 37°C.

L'essai est fait en double : une partie sur cellules infectées, pour la détermination de l'activité antivirale et l'autre partie sur cellules non infectées, pour déterminer la cytotoxicité du produit.

La première série est infectée par HIV (suspension de virus LAV-1-BRU Barré-Sinoussi et coll., Science, 220, 868 (1983) contenant environ 200 à 300 $TCID_{50}$) tandis que l'autre série reçoit 100 $\mu$l de milieu RPMI [contenant 10 % de sérum de veau foetal, 100 U/ml de pénicilline, 100 $\mu$g/ml de streptomycine et 2 umoles/ml de glutamine ($8 \times 10^4$ cellules par ml)].

La viabilité cellulaire est déterminée selon une modification de la technique décrite par R. PAUWELS et coll., J. Virol. Meth., 20, 309-321 (1988). Les puits, contenant encore les cellules (infectées ou non infectées), reçoivent 20 ul d'une solution de MTT (bromure de 4,5-diméthylthiazol-2-yl 2,5-diphényl tétrazolium) à 7 mg/ml dans du tampon phosphate isotonique. Pendant une période d'incubation de 3 heures, le MTT est converti en un sel de formazan (bleu) à l'intérieur des cellules vivantes et proportionnellement à leur nombre, alors que rien n'apparait dans les puits ne contenant plus que des cellules mortes. On ajoute alors 100 $\mu$l d'isopropanol (contenant 0,04 mole/l d'acide chlorydrique) et les microplaques sont agitées jusqu'à la solubilisation du bleu de formazan. L'absorption à 540 nm est lue avec un lecteur automatique de réactions ELISA en microplaques.

Dans ces conditions les cellules infectées sont lysées à environ 60-70 % par rapport aux cellules non infectées.

La formule :

$$\frac{\text{DO (cell. traitées et inf.) - DO (cell. non traitées et inf.)}}{\text{DO (cell. traitées et non inf.) - DO (cell. non traitées et inf.)}}$$

permet de calculer le taux de protection (%) du produit.

Les résultats figurent ci-après dans le tableau I.

2°) par dosage de la transcriptase inverse du virus VIH:

L'activité de la transcriptase inverse est mesurée directement sur le surnageant de culture selon la technique décrite par O. Schwartz et Coll., AIDS research and human retrovirus 4(6), 441-448 (1988)

L'activité transcriptase inverse (RT) est proportionnelle à la quantité de radioactivité acido-précipitable. Le % d'inhibition de la production virale est calculé au moyen de la formule :

$$1 - \frac{\text{cpm surnageants de cellules traitées et inf.}}{\text{cpm surnageants de cellules non traitées et inf.}}$$

Les résultats obtenus sont donnés dans le tableau I ci-après.

Tableau I

| MISE EN EVIDENCE DE L'ACTIVITE ANTIVIRALE | | | | | |
|---|---|---|---|---|---|
| Concentration µg/ml | Résultats au jour 13 | | (J13) | Dosage | de la RT |
| | Densité optique (DO) | | % de protection | Coups par mn (cpm) | % d'inhibit ion à J13 |
| | Cellules infectées | Cellules non infectées | | | |
| 0 | 122 | 480 | 0 | 37128 | 0 |
| 0,015 | 127 | 705 | 1 | 37095 | 0 |
| 0,05 | 149 | 677 | 5 | 35654 | 4 |
| 0,15 | 166 | 676 | 8 | 37310 | 0 |
| 0,5 | 204 | 649 | 16 | 31968 | 14 |
| 1,5 | 205 | 667 | 15 | 36633 | 1 |
| 5 | 432 | 729 | 51 | 32098 | 14 |
| 15 | 407 | 804 | 42 | 23085 | 38 |
| 50 | 79 | 85 | 116 | 1163 | 97 |

## CEM cl 13 JOUR 13

Les résultats précédents sont confirmés dans un second essai de dosage de la transcriptase inverse (Tableau II).

Tableau II

| Concentration µg/ml | Dosage de la RT | |
|---|---|---|
| | Coups par mn (cpm) | % d'inhibition à J13 |
| 0 | 34032 | 0 |
| 0,5 | 33268 | 2 |
| 1,5 | 30978 | 9 |
| 5 | 27917 | 18 |
| 15 | 13682 | 60 |
| 50 | 1227 | 96 |

Le produit défini précédemment manifeste également une activité anti-TNF (Tumour Necrosis Factor).
Le TNF est un cofacteur de l'activation du virus VIH notamment dans les cellules chroniquement infectées.

L'étude sur la production de TNF a été effectuée sur des cellules mononuclées de 4 donneurs sains, isolées à partir de sang périphérique sur un gradient de Ficoll. Après lavages les cellules sont mises en culture dans des plaques de microtitration à raison de $7,5.10^5$ cellules/puits dans 100 µl de milieu.

Les cellules sont incubées avec ou sans produit (concentrations échelonnées de 1,5 à 50 µg/ml) pendant 30 minutes à 37°C avant stimulation par 10 ng/ml de Phorbol 12-Myristate 13-Acétate (PMA).

Après une incubation de 24 heures à 37°C, sous une atmosphère contenant 5 % de gaz carbonique, les surnageants des différentes cultures ont été prélevés pour le dosage du TNF.

L'activité est estimée par la mesure de la cytotoxicité des surnageants vis à vis d'une lignée de fibroblastes murins sensibles aux TNF $\alpha$ et $\beta$. La cytotoxicité est estimée par la viabilité cellulaire mesurée par MTT et comparée à la cytotoxicité obtenue par une gage étalon de TNF $\alpha$ recombinant.

Cette expérience révèle une activité inhibitrice de la production de TNF dont la $CI_{50}$ est voisine de 10 µg/ml.

L'activité anti-TNF est particulièrement intéressante et permet une application au traitement des maladies dans le mécanisme desquelles intervient ce facteur. A titre d'exemple peuvent être cités notamment : choc septique, syndrome de détresse respiratoire, asthme et autres maladies chroniques respiratoires, maladies de résorption osseuse, réaction de greffon contre l'hôte, rejet d'allogreffe, fièvre et myalgies liées à des infections, SIDA et cachexie liée au SIDA, maladie de Crohn, colite ulcérante, malaria et forme cérébrale de la malaria.

## EFFET SUR LA PRODUCTION DE TNF

ETAT ACTUEL DE LA THERAPEUTIQUE

- L'histoire naturelle de l'infection par le virus de l'Immunodéficience Humaine (ou virus V.I.H) commence lorsque le virus pénètre dans un organisme humain en détruisant directement ou indirectement progressivement et principalement les lymphocytes T4. Cette destruction va déterminer l'apparition des manifestations cliniques (infections opportunistes) qui vont survenir lorsque leur taux est en général inférieur 200/mm3.

Les infections ne surviendront que plusieurs années après l'infestation initiale (4 à 10 années) et définissent les critères du S.I.D.A. (classification C.D.C. d'Atlanta). Dès leur apparition, la médiane statistique moyenne de survie oscille entre 9,6 et 13,5 mois en fonction des différentes données de la littérature. La médiane de survie de la pneumocystose principale infection opportuniste, varie de 10 à 18 mois selon les différentes séries publiées dans la littérature. La patiente dont l'histoire est décrite ci-dessus a déclaré sa pneumocystose il y a 19 mois et présente actuellement un taux de lymphocytes T4 d'un patient asymptomatique au stade 2 de la classification C.D.C.

En ce qui concerne le traitement de fond de l'infection V.I.H., la technique actuelle dispose de :
- la zidovudine, plus connue sous le nom d'A.Z.T. dont l'activité antirétrovirale in vitro a été démontrée en 1985. En 1986, les études de phase 2 réalisées par Margaret FISCHL chez 229 patients présentant une pneumocystose ou un ARC-Syndrome (pré-SIDA) a mis en évidence une survie à 6 mois significativement supérieure chez les patients traités par A.Z.T. (98 %) par rapport au groupe placebo (78 %). Ce produit a donc eu l'autorisation de mise sur le marché (A.M.M) en 1987 sous le nom de Retrovir (marque déposée). En 1988 le Pr DOURNON rapporte sur une étude exhaustive et prospective de 365 patients atteints de SIDA ou d'ARC-Syndrome des résultats non favorables. Dans cette étude, l'effet bénéfique de l'A.Z.T. ne dépasse pas 6 mois. A partir de cette date, les lymphocytes CD4 et la fréquence des infections opportunistes reviennent à leur valeur d'origine. La survie rejoint celle des patients non traités et ne modifie plus l'évolution naturelle de la maladie.

D'autres molécules telles que le d.d.I et d.d.C sont à l'heure actuelle en cours d'expérimentation. Les premières études de phase 1 réalisées par Yarchoan chez 92 patients (56 ARC-Syndrome et 36 S.I.D.A.) a montré une réponse favorable globale dans 29 % des cas (élévation des CD4 dans 25 %, diminution de l'antigénémie dans 46 %). Les études de phase 2 sont en cours d'évaluation, ce produit n'ayant pas encore obtenu l'A.M.M.

L'expérience des inventeurs de suivi d'environ 600 patients atteints de S.I.D.A. depuis 1983 n'a jamais montré d'évolution comparable à celle présentée chez cette patiente. Il est donc logique d'incriminer cette composition à base de buis comme étant l'élément actif favorable de cette évolution vue la chronologie de son suivi.

La présente invention concerne également les associations constituées d'un principe actif connu pour son activité antirétrovirus et d'une composition comprenant une plante de la famille des buxacées à l'état pur ou en présence d'excipients pharmaceutiquement acceptables.

## Revendications

1. Composition destinée au traitement préventif et/ou curatif du SIDA, ou des patients séropositifs pour le VIH, où pour diminuer le taux de TNF (Facteur de nécrose des tumeurs ou Tumor Necrosis Factor), caractérisée en ce qu'elle contient des feuilles de Buxus sempervirens, réduites en poudre, à l'état pur ou en combinaison avec des excipients inertes, pharmaceutiquement acceptables.

2. Composition selon la revendication 1, caractérisée en ce qu'elle contient en outre des parties de Buxus sempervirens choisies parmi la tige (bois), la racine et l'écorce de la racine, sous forme de poudre.

3. Utilisation d'au moins une partie d'une plante de la famille des buxacées pour la préparation d'un médicament destiné au traitement de déficit immunitaire.

4. Utilisation d'au moins une partie d'une plante de la famille des buxacées, pour la préparation d'un médicament destiné au traitement préventif et/ou curatif du SIDA ou de syndromes analogues, ou des patients séropositifs pour le VIH.

5. Utilisation selon la revendication 3 ou 4, caractérisée en ce que la plante de la famille des buxacées, à l'état pur ou en' combinaison avec des excipients inertes pharmaceutiquement acceptables, est associée avec un autre principe actif destiné au traitement du SIDA ou des patients séropositifs pour le VIH.

6. Utilisation d'une plante de la famille des buxacées pour la préparation d'un médicament destiné au traitement des affections dans lesquelles intervient directement ou indirectement le TNF (Tumor Necrosis factor).

7. Utilisation d'une plante de la famille des buxacées selon la revendication 6, pour la préparation d'un médicament destiné à diminuer le taux de TNF.

8. Utilisation selon l'une des revendications 3 à 7, caractérisée en ce qu'on utilise des feuilles broyées de Buxus sempervirens.

9. Utilisation selon l'une des revendications 3 à 8, caractérisée en ce qu'on utilise en outre une partie de Buxus sempervirens choisie parmi la tige (bois), la racine et l'écorce de la racine.

**10.** Utilisation selon l'une des revendications 3 à 9, caractérisée en ce que le médicament est essentiellement constitué d'alcaloïdes stéroïdiques spécifiques de la famille des buxacées dérivés du cycloarténol: cyclobuxine D et buxamine notamment.

**11.** Utilisation d'une composition selon l'une des revendications 1 ou 2 pour la fabrication d'un médicament destiné au traitement du syndrome d'immuno-dépression acquis ou des patients séropositifs pour le VIH ou d'un médicament destiné à diminuer le taux de TNF lors des maladies dans lesquelles ce facteur intervient.

**12.** Procédé d'obtention d'une composition selon l'une des revendications 1 ou 2, caractérisé en ce qu'il comporte les étapes suivantes:

- broyage d'au moins une partie d'une plante de la famille des buxacées, de manière à obtenir des particules d'environ 50 μm, le broyage étant fait de manière intermittente pour éviter tout échauffement susceptible d'altérer le ou les produits actifs,
- la poudre est alors mise dans des gélules qui sont stockées au sec.

**Claims**

1. Composition intended for the preventive and/or curative treatment of AIDS, or of HIV-seropositive patients, or for decreasing the level of TNF (tumour necrosis factor), characterized in that it contains Buxus sempervirens leaves reduced to powder, in the pure state or in combination with pharmaceutically acceptable, inert excipients.

2. Composition according to Claim 1, characterized in that it contains, in addition, parts of Buxus sempervirens chosen from the stem (wood), the root and the root bark, in powder form.

3. Use of at least one part of a plant of the family Buxaceae for the preparation of a medicament intended for the treatment of immune deficiency.

4. Use of at least one part of a plant of the family Buxaceae for the preparation of a medicament intended for the preventive and/or curative treatment of AIDS or of similar syndromes, or of HIV-seropositive patients.

5. Use according to Claim 3 or 4, characterized in that the plant of the family Buxaceae, in the pure state or in combination with pharmaceutically acceptable, inert excipients, is combined with another active principle intended for the treatment of AIDS or of HIV-seropositive patients.

6. Use of a plant of the family Buxaceae for the preparation of a medicament intended for the treatment of complaints in which TNF (tumour necrosis factor) is involved directly or indirectly.

7. Use of a plant of the family Buxaceae according to Claim 6, for the preparation of a medicament intended for decreasing the level of TNF.

8. Use according to one of Claims 3 to 7, characterized in that ground Buxus sempervirens leaves are used.

9. Use according to one of Claims 3 to 8, characterized in that a part of Buxus sempervirens, chosen from the stem (wood), the root and the root bark, is used in addition.

10. Use according to one of Claims 3 to 9, characterized in that the medicament consists essentially of steroid alkaloids specific to the family Buxaceae, derived from cycloartenol: cyclobuxine D and buxamine, in particular.

11. Use of a composition according to either of Claims 1 and 2, for the manufacture of a medicament intended for the treatment of acquired immune deficiency syndrome or of HIV-seropositive patients, or of a medicament intended for decreasing the level of TNF in diseases in which this factor is involved.

12. Method for obtaining a composition according to either of Claims 1 and 2, characterized in that it entails the following steps:

- grinding of at least one part of a plant of the family Buxaceae so as to obtain particles approximately 50 μm in size, the grinding being carried out intermittently in order to avoid any heating likely to degrade the active product or products,

- the powder is then placed in hard gelatin capsules, which are stored in a dry place.

**Patentansprüche**

1. Zusammensetzung für die Präventiv- und/oder Heilbehandlung von AIDS oder von HIV-seropositiven Patienten oder zur Verminderung des Gehaltes an TNF (Tumor-Nekrose-Faktor), dadurch gekennzeichnet, daß sie zu einem Pulver zerkleinerte Blätter des Gemeinen Buchsbaums (Buxus sempervirens) im reinen Zustand oder in Kombination mit pharmazeutisch akzeptablen inerten Exzipienten enthält.

2. Zusammensetzung nach Anspruch 1, dadurch gekennzeichnet, daß sie außerdem pulverförmige Teile des Gemeinen Buchsbaums (Buxus sempervirens), ausgewählt aus dem Stamm (Holz), der Wurzel und der Wurzelrinde, enthält.

3. Verwendung mindestens eines Teils einer Pflanze aus der Familie der Baxaceen zur Herstellung eines Arzneimittels für die Behandlung eines Immundefizits.

4. Verwendung mindestens eines Teils einer Pflanze aus der Familie der Buxaceen zur Herstellung eines Arzneimittels für die Präventiv- und/oder Heilbehandlung von AIDS oder analogen Syndromen oder von HIV-seropositiven Patienten.

5. Verwendung nach Anspruch 3 oder 4, dadurch gekennzeichnet, daß die Pflanze aus der Familie der Buxaceen in reinem Zustand oder in Kombination mit pharmazeutisch akzeptablen inerten Exzipienten mit einem anderen Wirkstoff für die Behandlung von AIDS oder von HIV-seropositiven Patienten kombiniert (assoziiert) ist.

6. Verwendung einer Pflanze aus der Familie der Buxaceen zur Herstellung eines Arzneimittels für die Behandlung von Erkrankungen, an denen direkt oder indirekt der TNF (Tumor-Nekrose-Faktor) beteiligt ist.

7. Verwendung einer Pflanze aus der Familie der Buxaceen nach Anspruch 6 zur Herstellung eines Arzneimittels für die Verminderung des Gehaltes an TNF.

8. Verwendung nach einem der Ansprüche 3 bis 7, dadurch gekennzeichnet, daß man gemahlene Blätter des Gemeinen Buchsbaums (Buxus sempervirens) verwendet.

9. Verwendung nach einem der Ansprüche 3 bis 8, dadurch gekennzeichnet, daß man außerdem einen Teil des Gemeinen Buchsbaums (Buxus sempervirens), ausgewählt aus dem Stamm (Holz), der Wurzel und der Wurzelrinde, verwendet.

10. Verwendung nach einem der Ansprüche 3 bis 9, dadurch gekennzeichnet, daß das Arzneimittel im wesentlichen aus spezifischen Steroid-Alkaloiden der Familie der Buxaceen besteht, die insbesondere Derivate von Cycloartenol, Cyclobuxin D und Buxamin sind.

11. Verwendung einer Zusammensetzung nach einem der Ansprüche 1 oder 2 zur Herstellung eines Arzneimittels für die Behandlung des AIDS-Syndroms oder von HIV-Virus-seropositiven Patienten oder eines Arzneimittels für die Verringerung des Gehaltes an TNF bei Erkrankungen, bei denen dieser Faktor eine Rolle spielt.

12. Verfahren zur Herstellung einer Zusammensetzung nach einem der Ansprüche 1 oder 2, dadurch gekennzeichnet, daß es die folgenden Stufen umfaßt:

    - Zerkleinern mindestens eines Teils einer Pflanze aus der Familie der Buxaceen in der Weise, daß man Teilchen mit einer Größe von etwa 50 μm erhält, wobei das Zerkleinern intermittierend durchgeführt wird, um jede Erwärmung zu vermeiden, die den Wirkstoff oder die Wirkstoffe verändern könnte, und
    - anschließendes Einfüllen des Pulvers in Kapseln, die trocken gelagert werden.